(19) Europäisches Patentamt
European Patent Office
Office européen des brevets

(11) Veröffentlichungsnummer: **0 240 828**
**A1**

(12) **EUROPÄISCHE PATENTANMELDUNG**

(21) Anmeldenummer: **87104269.3**

(51) Int. Cl.4: **C07D 211/90** , A61K 31/44

(22) Anmeldetag: **23.03.87**

Die Bezeichnung der Erfindung wurde geändert (Richtlinien für die Prüfung im EPA, A-III, 7.3).

(30) Priorität: **27.03.86 CH 1263/86**

(43) Veröffentlichungstag der Anmeldung:
**14.10.87 Patentblatt 87/42**

(84) Benannte Vertragsstaaten:
**AT BE CH DE ES FR GB GR IT LI LU NL SE**

(71) Anmelder: **Byk Gulden Lomberg Chemische Fabrik GmbH**
**Byk-Gulden-Strasse 2**
**D-7750 Konstanz(DE)**

(72) Erfinder: **Amschler, Hermann**
**Hohenhewenstrasse 19**
**D-7760 Radolfzell(DE)**
Erfinder: **Kohl, Bernhard**
**Heinrich-von-Tettingen-Strasse 35a**
**D-7750 Konstanz 19(DE)**
Erfinder: **Eltze, Manfrid**
**Schützenstrasse 20**
**D-7750 Konstanz(DE)**
Erfinder: **Flockerzi, Dieter**
**Ackerweg 26**
**D-7753 Allensbach(DE)**
Erfinder: **Klemm, Kurt**
**Im Weinberg 2**
**D-7753 Allensbach(DE)**
Erfinder: **Kolassa, Norbert**
**Lerchenweg 12**
**D-7750 Konstanz(DE)**
Erfinder: **Sanders, Karl**
**Felchengang 23**
**D-7750 Konstanz(DE)**
Erfinder: **Schudt, Christian**
**Hoheneggstrasse 102**
**D-7750 Konstanz(DE)**
Erfinder: **Ulrich, Wolf-Rüdiger**
**Hebelstrasse 3**
**D-7750 Konstanz(DE)**

(54) **Optisch aktive 1,4-Dihydropyridinverbindung.**

(57) Die Erfindungs betrifft den (+)-1,4-Dihydro-2,6-dimethyl-4-(3-nitrophenyl)-pyridin-3,5-dicarbonsäure-3-methyl-5-[3-(4,4-diphenylpiperidyl-1)-propyl]-ester und seine Salze sowie ein Verfahren zu seiner Herstellung.

EP 0 240 828 A1

# Neue optisch aktive Verbindung

## Anwendungsgebiet der Erfindung

Die Erfindung betrifft eine neue optisch aktive Verbindung sowie ein Verfahren zu ihrer Herstellung. Die neue Verbindung wird in der pharmazeutischen Industrie zur Herstellung von Arzneimitteln eingesetzt.

## Bekannter technischer Hintergrund

Es ist bekannt, daß bestimmte, in 4-Position substituierte 1,4-Dihydropyridinderivate pharmakologisch nützliche Eigenschaften aufweisen. Ferner ist bekannt, daß diese 1,4-Dihydropyridinderivate - sofern sie in den Positionen 2 und 6 und/oder in den Positionen 3 und 5 unterschiedlich (unsymmetrisch) substituiert sind - in der Position 4 ein Chiralitätszentrum aufweisen. Außerdem ist bekannt, daß die pharmakologischen Eigenschaften der 1,4-Dihydropyridine von der absoluten Konfiguration in der 4-Position beeinflußt werden können. Überraschenderweise wurde nun gefunden, daß das unten näher beschriebene reine Enantiomere eines chiralen Dihydropyridins, dessen Racemat in der Europäischen Patentanmeldung 176 956 beschrieben ist, eine unerwartet starke pharmakologische Wirksamkeit aufweist, durch die es sich von den bisher bekannten enantiomer reinen Dihydropyridinen in überraschender Weise unterscheidet.

## Beschreibung der Erfindung

Gegenstand der Erfindung ist das das linear polarisierte Licht der Wellenlänge 589 nm in (+)-Richtung drehende Enantiomere des 1,4-Dihydro-2,6-di-methyl-4-(3-nitrophenyl)-pyridin-3,5-dicarbonsäure-3-methyl-5-[3-(4,4-diphenylpiperidyl-1)-propyl]-esters, also der (+)-1,4-Dihydro-2,6-dimethyl-4-(3-nitrophenyl)-pyridin-3,5-dicarbonsäure-3-methyl-5-[3-(4,4-diphenylpiperidyl-1)-propyl]-ester, und seine Salze.

Ohne Berücksichtigung der absoluten Konfiguration in der 4-Position des 1,4-Dihydropyridins wird die erfindungsgemäße Verbindung durch die folgende Formel beschrieben:

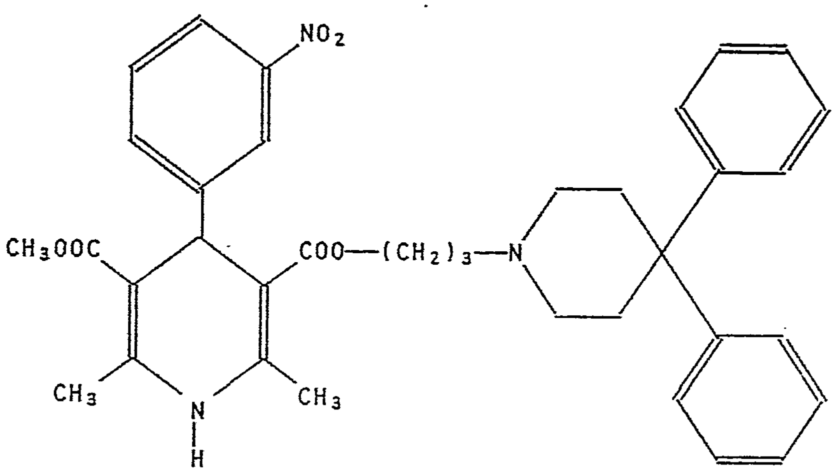

(+)

Als Salze kommen alle Salze mit Säuren in Betracht. Besonders erwähnt seien die pharmakologisch verträglichen Salzen der in der pharmazeutischen Industrie üblicherweise verwendeten anorganischen und organischen Säuren. Pharmakologisch unverträgliche Salze, die beispielsweise bei der Herstellung der erfindungsgemäßen Verbindung im industriellen Maßstab als Verfahrensprodukte zunächst anfallen können, werden durch dem Fachmann bekannte Verfahren in pharmakologisch verträgliche Salze übergeführt. Als solche eignen sich beispielsweise wasserlösliche und wasserunlösliche Säureadditionssalze, wie das Hydrochlorid, Hydrobromid, Hydroiodid, Phosphat, Nitrat, Sulfat, Acetat, Citrat, Gluconat, Benzoat, Hibenzat,

Fendizoat, Butyrat, Sulfosalicylat, Maleat, Laurat, Malat, Fumarat, Succinat, Oxalat, Tartrat, Amsonat, Metembonat, Stearat, Tosilat, 2-Hydroxy-3-naphthoat, 3-Hydroxy-2-naphthoat oder Mesilat, aber auch Salze mit Bumetanid, Furosemid, Azosemid, Galosemid, Besunid, Piretanid, Etacrynsäure, Tienilinsäure oder 4-Chlor-sulfamoyl-benzoesäure.

Gegenstand der Erfindung ist weiterhin ein Verfahren zur Herstellung der erfindugnsgemäßen Verbindung und ihrer Salze. Das Verfahren ist dadurch gekennzeichnet, daß man (±)-1,4-Dihydro-2,6-dimethyl-4-(3-nitrophenyl)-pyridin-3,5-dicarbonsäure-3-methyl-5[3-(4,4-diphenylpiperidyl-1)-propyl]-ester mit einer enantiomer reinen optisch aktiven Säure umsetzt, die erhaltenen diastereomeren Salze trennt, aus dem gewünschten diastereo meren Salz den (+)-1,4-Dihydro-2,6-dimethyl-4-(3-nitrophenyl)-pyridin-3,5-dicarbonsäure-3-methyl-5-[3-(4,4-diphenylpiperidyl-1)-propyl]-ester durch Zugabe von Base freisetzt und ihn gewünschtenfalls anschließend in ein Salz überführt.

Als optisch aktive Säuren seien beispielsweise die Di-0,0′-p-toluoylweinsäure oder insbesondere die Di-0,0′-benzoylweinsäure genannt. Als Trennverfahren eignet sich vorzugsweise die Umkristallisation.

Die mit Hilfe dieser Methoden getrennten, konfigurativ einheitlichen diastereomeren Salze werden durch Zugabe vorzugsweise anorganischer Basen, wie z.B. Ammoniak, oder mit Hilfe von basischen Ionenaustauschern in die optisch aktive, enantiomer reine erfindungsgemäße Verbindung überführt.

Die Isolierung und Reinigung der erfindungsgemäßen Verbindung erfolgt in an sich bekannter Weise z. B. derart, daß man das Lösungsmittel im Vakuum abdestilliert und den erhaltenen Rückstand aus einem geeigneten Lösungsmittel umkristallisiert oder einer der üblichen Reinigungsmethoden, wie beispielsweise der Säulenchromatographie an geeignetem Trägermaterial, unterwirft.

Säureadditionssalze erhält man durch Auflösen der freien Base in einem geeigneten Lösungsmittel, z.B. in einem chlorierten Kohlenwasserstof, wie Methylenchlorid oder Chloroform, oder einem niedermolekularen aliphatischen Alkohol (Ethanol, Isopropanol), das die gewünschte Säure enthält, oder dem die gewünschte Säure anschließend zugegeben wird.

Die Salze werden durch Filtrieren, Umfällen, Ausfällen mit einem Nichtlösungsmittel für das Anlagerungssalz oder durch Verdampfen des Lösungsmittels gewonnen.

Erhaltene Salze können durch Alkalisierung, z.B. mit wäßriger Ammoniaklösung, in die freien Basen umgewandelt werden, welche wiederum in Säureadditionssalze übergeführt werden können. Auf diese Weise lassen sich pharmakologisch nicht verträgliche Säureadditionssalze in pharmakologisch verträgliche Säureadditionssalze umwandeln.

Die Herstellung des der erfindungsgemäßen Verbindung zugrundeliegenden Racemates, also die Herstellung der 1:1-Mischung mit dem entsprechenden (-)-Enantiomeren, ist in den Beispielen beschrieben.

Die folgenden Herstellungsbeispiele sollen die Erfindung näher erläutern, ohne sie einzuschränken. Schmp. bedeutet Schmelzpunkt, h steht für Stunden, Kp. steht für Siedepunkt, Zers. bedeutet Zersetzung.

## Beispiele

1. (+)-1,4-Dihydro-2,6-dimethyl-4-(3-nitrophenyl)-pyridin-3,5-dicarbonsäure-3-methyl-5=[3-(4,4-diphenylpiperidyl-1)-propyl]-ester-hydrochlorid

4,53 g 3-Nitrobenzaldehyd, 3,45 g 3-Aminocrotonsäuremethylester und 11,38 g Acetessigsäure-[3-(4,4-diphenylpiperidyl-1)-propyl]-ester in 100 ml 2-Propanol werden über Nacht unter Rückfluß zum Sieden erhitzt. Die abgekühlte Lösung wird zur Trockne eingeengt und der verbleibende Rückstand über eine Kieselgelsäule mit Essigsäureethylester als Eluens chromatographiert. Die einheitlichen Produktfraktionen hinterlassen nach dem Einengen einen festen aufgeschäumten Rückstand, der in Methanol gelöst und mit etherischer Salzsäure versetzt wird. Die Lösung wird eingeengt, der verbleibende feste Rückstand in wenig Methanol aufgenommen und die Titelsubstanz durch Zugabe von Petrolether ausgefällt. Scmp.: ab 135°C (Zersetzung); Ausbeute: 9,3 g.

Die freie Base der Titelverbindung, die für die Enantiomerentrennung benötigt wird, erhält man, wenn der nach dem Einengen des Kondensationsansatzes verbleibende aufgeschäumte feste Rückstand in wenig Methylenchlorid aufgenommen wird; nach Zusatz von Diisopropylether bis zur bleibenden feinen Trübung kristallisiert die Base nach Stehen im Kühlschrank in feinen Plättchen aus. Schmp.: 145-147°C.

Die Ausgangsverbindungen werden wie folgt erhalten:

a) Acetessigsäure-[3-(4,4-diphenylpiperidyl-1)-propyl]-ester

23,6 g 3-(4,4-Diphenylpiperidyl-1)-propanol werden in 100 ml abs. Toluol gelöst und unter Rühren mit 16 ml einer 50%igen Diketenlösung in Aceton versetzt. Nach mehrtägigem Stehen bei Raumtemperatur (DC-Kontrolle) wird der Ansatz eingeengt und der Rückstand im Hochvakuum getrocknet. Das verbleibende hellgelbe, zähe Öl wird ohne weitere Reinigung für die nächste Stufe eingesetzt.

b) 3-(4,4-diphenylpiperidyl-1)-propanol

40 g 4,4-Diphenylpiperidin, 24,7 g 3-Brompropanol, 116,4 g pulverisiertes Kaliumcarbonat und ca. 1 g Kaliumiodid werden in 500 ml eines 1:1 Gemisches aus Dioxan und 1-Butanol ca. 48 Stunden unter Rückfluß und kräftigem Rühren zum Sieden erhitzt. Nach dem Abkühlen wird filtriert, das Filtrat eingeengt. Der ölige Rückstand wird in Essigsäureethylester aufgenommen und die Lösung nochmals filtriert. Nach dem Einengen des Filtrats zur Trockne erhält man das Produkt als gelblichen öligen Rückstand, der langsam wachsartig fest wird. Ausbeute: 44,8 g. Mit etherischer Salzsäure erhält man das Hydrochlorid, das in 2-Propanol umkristallisiert wird. Schmp.: 226-7°C.

Alternativ wird die Ausgangsverbindung b) erhalten, wenn 352 g 4,4-Diphenylpiperidin, 128 g Natriumhydroxidgranulat, 2,5 l Methylenchlorid, 500 ml Wasser, 218 g 3-Brom-1-propanol und katalytische Mengen eine Phasentransferkatalysators (z.B. Benzyltrimethylammoniumchlorid) 10 Stunden unter Rückfluß zum Sieden erhitzt werden. Die abgetrennte organische Phase wird mit Wasser gewaschen, die gesammelten Wasserphasen werden mit Methylenchlorid extrahiert. Nach dem Trocknen der vereinigten organischen Phasen mit Natriumsulfat wird die klare, bräunliche Lösung zur Trockne eingedampft. Der harzige braune Rückstand wird in 4,5 l siedendem Petrolether (Siedebereich 100-140°C) aufgenommen, die Lösung heiß von unlöslichem Rückstand abfiltriert und abgekühlt. Nach Stehenlassen über Nacht erhält man die Ausgangsverbindung als freie Base in farblosen, groben Kristallen. Schmp.: 97°C. Ausbeute: 303 g.

2. (+)-1,4-Dihydro-2,6-dimethyl-4-(3-nitrophenyl)-pyridin-3,5-dicarbon-säure-3-methyl-5-[3-(4,4-diphenylpiperidyl-1)-propyl]-ester-(+)-di-0,0'-benzoyltartrat

18,2 g (±)-1,4-Dihydro-2,6-dimethyl-4-(3-nitrophenyl)-pyridin-3,5-dicarbonsäure-3-methyl-5-[3-(4,4-diphenylpiperidyl-1)-propyl]-ester und 11,29 g käufliches D-(+)-Di-0,0'-benzoylweinsäure-hydrat (im vorliegenden Beispiel mit dem spezifischen Drehwert $[\alpha]_D^{23}$ = + 106° (c = 5, Ethanol) werden in 100 ml Methanol zusammen gelöst. Die klare Lösung wird anschließend zur Trockene eingeengt und der erhaltene kristalline Rückstand in einem Gemisch aus 280 ml Chloroform und 20 ml Methanol in der Siedehitze umkristallisiert. Nach dem langsamen Erkalten der Lösung erhält man ein erstes Kristallisat von Schmp.: 143-144°C; Ausbeute: ca. 14 g {(grobe leicht gelbliche Nadeln, $[\alpha]_D^{23}$ = +48° (c = 5, Ethanol)]. Erneutes Umkristallisieren des ersten Kristallisates in Chloroform/Methanol ergibt ein zweites Kristallisat von Schmp.: 145-146°C; Ausbeute: ca. 12 g {(grobe leicht gelbliche Nadeln, $[\alpha]_D^{23}$ = +49° (c = 5, Ethanol)}. Weiteres Umkristalisieren des zweiten Kristallisates in Chloroform/Methanol ergibt ein drittes Kristallisat von Schmp. 147-148°C; Ausbeute: ca. 9 g {grobe leicht gelbliche Nadeln, $[\alpha]_D^{23}$ = +50 (c = 5, Ethanol)}.

Bei Verwendung von D-(+)-Di-0,0'-benzoylweinsäure mit höherer optischer Reinheit erhöhen sich die spezifischen Drehwerte der erhaltenen Salze entsprechend.

3. (+)-1,4-Dihydro-2,6-dimethyl-4-(3-nitrophenyl)-pyridin-3,5-dicarbon-säure -3-methyl-5-[3-(4,4-diphenylpiperidyl-1)-propyl]-ester-hydrochlorid

4,84 g des dritten Kristallisates aus Beispiel 2 werden in 100 ml Dichlormethan gelöst und mit je 50 ml halbkonzentrierter wäßriger Ammoniak-Lösung dreimal extrahiert. Die wäßrigen Phasen werden jeweils mit 50 ml Dichlormethan erneut extrahiert und die vereinigten organischen Phasen zweimal mit je 100 ml Waser gewaschen und anschließend über Natriumsulfat getrocknet. Nach dem vollständigen Einengen der Dichlormethanlösung löst man den als festen Rückstand erhaltenen (+)-1,4-Dihydro-2,6-dimethyl-4-(3-nitrophenyl)-pyridin-3,5-dicarbonsäure-3-methyl-5-[3-(4,4-diphenylpiperidyl-1)-propyl]-ester in 5 ml Methanol

und versetzt die Lösung mit etherischer Salzsäure. Die Lösung wird erneut vollständig zur Trockene eingeengt und der erhaltene feste Rückstand in 9 ml Dioxan heiß gelöst. Nach Erkalten der Lösung erhält man die Titelverbindung als feine Nadelbüschel. Schmp.: ab 142°C bis 162°C (langsames Zerfließen). Ausbeute: 3,1 g. $[\alpha]_D^{22}$ = + 13,9° (c = 1, Methanol).

Bei Verwendung von D-(+)-Di-0,0'-benzoylweinsäure mit höherer optischer Reinheit erhöht sich der spezifische Drehwert der Titelverbindung entsprechend.

## Gewerbliche Anwendbarkeit

Die erfindungsgemäße Verbindung und ihre Salze besitzen wertvolle Eigenschaften, die sie gewerblich verwertbar machen. Sie stellen insbesondere wirksame Vasodilatoren mit coronartherapeutischen Eigenschaften dar. Die pharmakologische Wirksamkeit der erfindungsgemäßen Verbindung zeigt sich insbesondere in einer langsam eintretenden, starken und langanhaltenden Blutdrucksenkung. Darüberhinaus besitzt die erfindungsgemäße Verbindung hemmende Wirkung auf den Calciumeinstrom sowie fördernde Wirkung auf den Kaliumausstrom von Zellen, glattmuskulär relaxierende und peripher, coronar, cerebral und renal gefäßerweiternde sowie salidiuretische, antithrombotische, antiarteriosklerotische und günstige hämorheologische Eigenschaften.

In ihrer ausgezeichneten Wirksamkeit, die gepaart ist mit einer geringen Toxizität und dem Fehlen wesentlicher Nebenwirkungen, unterscheidet sich die erfindungsgemäße Verbindung in überraschender und vorteilhafter Weise von den Verbindungen des Standes der Technik. Insbesondere unterscheidet sich die erfindungsgemäße Verbindung in überraschender Weise auch von ihrem korrespondierenden Racemat, den (±) -1,4-Dihydro-2,6-dimethyl-4-(3-nitrophenyl)-pyridin-3,5-dicarbonsäure-3-methyl-5-[3-(4,4-diphenylpiperidyl-1)-propyl]-ester sowie dem entsprechenden (-)-Enantiomeren.

Als vorteilhafte Eigenschaften der erfindungsgemäßen Verbindung sind beispielsweise zu nennen: das Ausmaß der Blutdrucksenkung, das lange Anhalten der Blutdrucksenkung, die gute Steuerbarkeit der Blutdrucksenkung, die überraschend geringe und bei wiederholter Gabe verschwindende Herzfrequenzsteigerung, die ausgezeichnete Bioverfügbarkeit, die große therapeutische Breite, das Fehlen zentraler Nebenwirkungen, das Fehlen kinetischer Interaktionen mit anderen Substanzen, das Ausbleiben einer Toleranzentwicklung, die ausgewogenen physikalischen Eigenschaften und die große Stabilität.

Die ausgezeichnete Wirksamkeit der erfindungsgemäßen Verbindung und ihrer Salze gestattet ihren Einsatz in der Humanmedizin, wobei als Indikation insbesondere primäre (essentielle) und sekundäre, arterielle und pulmonale Hypertonien aller Schweregrade, koronare Herzkrankheiten (Koronarinsuffizienz, Angina Pectoris, Myocardinfarkt etc.), periphere und cerebrale Zirkulationsstörungen (Gehirnschlag, temporäre cerebrale Durchblutungsstörungen, Migräne, Schwindel, renale Arterienverengung etc.), hypertrophe Kardiomyopathie, Herzinsuffizienz, Krankheiten, die auf einer erhöhten Wasser-und Natriumretention beruhen und Krankheiten, die auf einem erhöhten Calciumeinstrom beruhen, wie z.B. Spasmen glattmuskulärer Organe (Atemwege, Gastrointestnaltrakt, Urogenitaltrakt etc.) sowie Arrhythmie, Arteriosklerose und Zellschädigungen verschiedener Genese (z.B. Hypoxie) in Betracht kommen.

Ein weiterer Gegenstand der Erfindung ist daher ein Verfahren zur Behandlung von Säugetieren, insbesondere Menschen, die an einer der obengenannten Krankheiten erkrankt sind. Das Verfahren ist dadurch gekannzeichnet, daß man dem erkrankten Individuum eine therapeutisch wirksame und pharmakologisch verträgliche Menge der erfindungsgemäßen Verbindung oder ihrer pharmakologisch verträglichen Salze verabreicht.

Gegenstand der Erfindung ist außerdem die erfindungsgemäße Verbindung und ihre pharmakologisch verträglichen Salze zur Anwendung bei der Behandlung der genannten Krankheiten.

Ebenso umfaßt die Erfindung die Verwendung der erfindungsgemäße Verbindung und ihre pharmakologisch verträglichen Salze bei der Herstellung von Arzneimitteln, die zur Bekämpfung der genannten Krankheiten eingesetzt werden.

Ein weiterer Gegenstand der Erfindung sind Arzneimittel, die die erfindungsgemäße Verbindung und/oder ihre pharmakologisch verträglichen Salze enthalten.

Die Arzneimittel werden nach an sich bekannten, dem Fachmann geläufigen Verfahren hergestellt. Als Arzneimittel wird die erfindungsgemäße pharmakologisch wirksame Verbindung und/oder ihre pharmakologisch verträglichen Salze (=Wirkstoffe) entweder als solche, oder vorzugsweise in Kombination mit geeigneten pharmazeutischen Hilfsstoffen in Form von Tabletten, Dragees, Kapseln, Suppositorien, Pflastern (z.B. als TTS), Emulsionen, Suspensionen, Aerosolen, Sprays, Salben, Cremes, Gelen oder Lösungen eigesetzt, wobei der Wirkstoffgehalt vorteilhafterweise zwischen 0,1 und 95% beträgt.

Welche Hilfsstoffe für die gewünschten Arzneimittelformulierungen geeignet sind, ist dem Fachmann aufgrund seines Fachwissens geläufig. Neben Lösemitteln, Gelbildnern, Suppositoriengrundlagen, Tabletten, Hilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler, Farbstoffe oder insbesondere Permeationspromotoren und Komplexbildner (z.B. Cyclodextrine) verwendet werden.

Die Wirkstoffe können oral, rektal, per inhalationem oder parenteral (insbesondere perlingual, intravenös oder percutan) appliziert werden.

Im allgemeinen hat es sich in der Humanmedizin als vorteilhaft erwiesen, den oder die Wirkstoffe bei oraler Gabe in einer Tagesdosis von etwa 0,01 bis etwa 10, vorzugsweise 0,05 bis 5 mg/kg Körpergewicht, gewünschtenfalls in Form mehrerer, vorzugsweise 1 bis 4 Einzelgaben zur Erzielung des gewünschten Ergebnisses zu verabreichen. Bei einer parenteralen Behandlung können ähnliche bzw. (insbesondere bei der intravenösen Verabreichung der Wirkstoffe) in der Regel niedrigere Dosierungen zur Anwendung kommen. Bei einschleichender Dosierung wird zu Beginn der Behandlung eine geringere Dosis verabreicht, dann langsam auf eine höhere Dosis übergegangen. Nach Erreichen des gewünschten Therapieerfolges wird wieder auf eine niedrigere Dosis zurückgegangen.

Die Festlegung der jeweils erforderlichen optimalen Dosierung und Applikationsart der Wirkstoffe kann durch jeden Fachmann aufgrund seines Fachwissens leicht erfolgen.

Sollen die erfindungsgemäße Verbindung und/oder ihre pharmakologisch verträglichen Salze zur Behandlung der genannten Krankheiten eingesetzt werden, so können die pharmazeutischen Zubereitungen auch einen oder mehrere andere pharmakologisch aktive Bestandteile anderer Arzneimittelgruppen, wie andere Vasodilatoren, Antihypertensiva, alpha-1-Rezeptorenblocker, alpha-2-Rezeptorstimulatoren, beta-1-Rezeptorenblocker, beta-2-Rezeptorstimulatoren, ACE-Hemmstoffe, Nitroverbindungen, Cardiotonika, Diuretika, Saluretika, Alkaloide, Analgetika, Lipidsenker, Antikoagulan tien, Anticholinergika, Methylxanthine, Antiarrhythmika, Antihistaminika, Dopaminstimulatoren, Serotonin-Rezeptorenblocker etc., wie Nifedipin, Dihydralazin, Prazosin, Clonidin, Atenolol, Labetalol, Fenoterol, Captopril, Isosorbiddinitrat, Digoxin, Milrinon, Mefrusid, Clopamid, Spironolacton, Chlorthalidon, Furosemid, Polythiazid, Hydrochlorothiazid, Reserpin, Dihydroergocristin, Rescinnamin, Rauwolfia-Gesamtalkaloide, Acetylsalicylsäure, Bezafibrat, Warfarin, Atropin, Theophyllin, Lidocain, Astemizol, Bromocryptin, Ketanserin etc. enthalten.

## Pharmakologie

Die antihypertensive Wirksamkeit der erfindungsgemäßen Verbindung kann am Modell der spontan hypertonen Ratte nachgewiesen werden.

Zur Bestimmung der antihypertensiven Wirkung wird die Verbindung in den angegebenen Dosen an je 6 bzw. 12 wachen männlichen Ratten (Stamm SHR/N/Ibm/Bm, 350-400 g) mit genetisch bedingtem Hochdruck (arterieller Mitteldruck 160-200 mmHg) akut verabfolgt, und zwar intravenös durch Injektion über einen Katheter in die Vena jugularis bzw. intragastral mittels Schlundsonde. Die Messung des Blutdruckes erfolgt fortlaufend bis zu 6 Stunden nach Substanzgabe über einen in die Aorta abdominalis verlegten Katheter und konventionelle Druckregistrierung mittels piezo-elektrischem Druckwandler. Blutdruckwerte werden in einem engen Zeitraster bis zu 60 Min. nach Substanzgabe erfaßt (1, 3, 5, 10, 15, 30, 45, 60 Min. nach i.v.-Gabe bzw. 5, 10, 20, 30, 40, 50 und 60 Min. nach p.o.-Gabe) und anschließend in stündlichem Intervall bis 360 Min. nach Substanzgabe. Als Maß für die Veränderung des Blutdruckes infolge Substanzgabe wird anschließend die AUC (area under the curve) ermittelt und zwar a) für die Teilzeitperiode 0-60 Min. und b) 60-360 Min. nach Substanzgabe.

Die Tabellen I und II geben für die erfindungsgemäße Verbindung (= Verbindung Nr. 1), für das dazugehörige Racemat (= Verbindung Nr. 2) sowie für das korrespondierende (-)-Enantiomere (=Verbindung Nr. 3) die mittlere Senkung des Blutdruckes (mmHg) in den beiden genannten Teilzeitperioden nach i.v. (Tabelle I) bzw. p.o. Substanzgabe (Tabelle II) an.

Tabelle I

Mittlere Senkung des Blutdruckes genetisch hypertoner Ratten (n = Anzahl) nach akuter i.v.-Applikation:

| Verbindung Nr. | Dosis µmol/kg | n | AUC (mmHg) 0 - 60 Min. | AUC (mmHg) 60 - 360 Min. |
|---|---|---|---|---|
| 1 | 0,03 | 6 | - 33 | - 14 |
| 1 | 0,1 | 12 | - 70 | - 39 |
| 2 | 0,03 | 6 | - 12 | + 1 |
| 2 | 0,1 | 6 | - 58 | - 25 |
| 3 | 1,0 | 6 | - 10 | + 4 |
| 3 | 3,0 | 6 | - 30 | + 1 |

Tabelle II

Mittlere Senkung des Blutdruckes genetisch hypertoner Ratten (n = Anzahl) nach akuter p.o.-Applikation (in Lösung):

| Verbindung Nr. | Dosis µmol/kg | n | AUC (mmHg) 0 - 60 Min. | AUC (mmHg) 60 - 360 Min. |
|---|---|---|---|---|
| 1 | 1 | 12 | - 16 | - 34 |
| 1 | 3 | 6 | - 44 | - 79 |
| 2 | 1 | 6 | - 7 | - 31 |
| 2 | 3 | 6 | - 27 | - 47 |
| 3 | 10 | 6 | - 12 | - 6 |
| 3 | 30 | 6 | - 4 | - 19 |

## Ansprüche

1. (+)-1,4-Dihydro-2,6-dimethyl-4-(3-nitrophenyl)-pyridin-3,5-dicarbonsäure-3-methyl-5-[3-(4,4-diphenylpiperidyl-1)-propyl]-ester und seine Salze.

2. (+)-1,4-Dihydro-2,6-dimethyl-4-(3-nitrophenyl)-pyridin-3,5-dicarbonsäure-3-methyl-5-[3-(4,4-diphenylpiperidyl-1)-propyl]-ester-hydrochlorid.

3. Verfahren zur Herstellung von (+)-1,4-dihydro-2,6-dimethyl-4-(3-nitrophenyl)-pyridin-3,5-dicarbonsäure-3-methyl-5-[3(4,4-diphenylpiperidyl-1)-propyl]-ester und seinen Salzen, dadurch gekennzeichnet, daß man (±)-1,4-Dihydro-2,6-dimethyl-4-(3-nitrophenyl)-pyridin-3,5-dicarbonsäure-3-methyl-5-[3-(4,4-diphenylpiperidyl-1)-propyl]-ester mit einer enantiomer reinen optisch aktiven Säure umsetzt, die erhaltenen Diastereomeren trennt, aus dem gewünschten Diastereomeren den (+)-1,4-Dihydro-2,6-dimethyl-4-(3-nitrophenyl)-pyridin-3,5-dicarbonsäure-3-methyl-5-[3-(4,4-diphenylpiperidyl-1)-propyl]-ester durch Zugabe von Base freisetzt und ihn gewünschtenfalls anschließend in ein Salz überführt.

4. Verfahren nach Anspruch 3, dadurch gekennzeichnet, daß die enantiomer reine optisch aktive Säure D-(+)-Di-0,0'-benzoylweinsäure ist.

5. Arzneimittel enthaltend (+)-1,4-Dihydro-2,6-dimethyl-4-(3-nitrophenyl)-pyridin-3,5-dicarbonsäure-3-methyl-5-[3-(4,4-diphenylpiperidyl-1)-propyl]-ester und/oder seine pharmakologisch verträglichen Salze.

6. (+)-1,4-Dihydro-2,6-dimethyl-4-(3-nitrophenyl)-pyridin-3,5-dicarbonsäure-3-methyl-5-[3-(4,4-diphenylpiperidyl-1)-propyl]-ester und seine pharmakologisch verträglichen Salze zur Anwendung bei der Behandlung und/oder Prophylaxe von Hypertonie, koronaren Herzkrankheiten, peripheren und cerebralen Zirkulationsstörungen und/oder Krankheiten, die auf einer erhöhten Wasser-oder Natriumretention beruhen.

7. (+)-1,4-Dihydro-2,6-dimethyl-4-(3-nitrophenyl)-pyridin-3,5-dicarbonsäure-3-methyl-5-[3-(4,4-diphenylpiperidyl-1)-propyl]-ester und seine pharmakologisch verträglichen Salze zur Anwendung bei der Behandlung von Krankheiten.

8. Verwendung von (+)-1,4-Dihydro-2,6-dimethyl-4-(3-nitrophenyl)-pyridin-3,5-dicarbonsäure-3-methyl-5-[3-(4,4-diphenylpiperidyl-1)-propyl]-ester und seinen pharmakologisch verträglichen Salze zur Herstellung von Arzneimitteln für die Behandlung und/oder Prophylaxe von Hypertonie, koronaren Herzkrankheiten, peripheren und cerebralen Zirkulationsstörungen und/oder Krankheiten, die auf einer erhöhten Wasser-oder Natriumretention beruhen.

Patentansprüche für die folgenden Vertragsstaaten: AT, ES, GR.

1. Verfahren zur Herstellung von (+)-1,4-Dihydro-2,6-dimethyl-4-(3-nitrophenyl)-pyridin-3,5-dicarbonsäure-3-methyl-5-[3-(4,4-diphenylpiperidyl-1)-propyl]-ester und seinen Salzen, dadurch gekannzeichnet, daß man (±)-1,4-Dihydro-2,6-dimethyl-4-(3-nitrophenyl)-pyridin-3,5-dicarbonsäure-3-methyl-5-[3-(4,4-diphenylpiperidyl-1)-propyl]-ester mit einer enantiomer reinen optisch aktiven Säure umsetzt, die erhaltenen Diastereomeren trennt, aus dem gewünschten Diastereomeren den (+)-1,4-Dihydro-2,6-dimethyl-4-(3-nitrophenyl)-pyridin-3,5-dicarbonsäure-3-methyl-5-[3-(4,4-diphenylpiperidyl-1)-propyl]-ester durch Zugabe von Base freisetzt und ihn gewünschtenfalls anschließend in ein Salz überführt.

2. Verfahren nach Anspruch 1, dadurch gekennzeichnet, daß die enantiomer reine optisch aktive Säure D-(+)-Di-0,0'-benzoylweinsäure ist.

3. Verfahren nach Anspurch 1, dadurch gekennzeichnet, daß der (+)-1,4-Dihydro-2,6-dimethyl-4-(3-nitrophenyl)-pyridin-3,5-dicarbonsäure-3-methyl-5-[3-(4,4-diphenylpiperidyl-1)-propyl]-ester anschließend in das Hydrochlorid überführt wird.

4. Verfahren zur Herstellung von Arzneimitteln enthaltend (+)-1,4-Dihydro-2,6-dimethyl-4-(3-nitrophenyl)-pyridin-3,5-dicarbonsäure-3-methyl-5-[3-(4,4-diphenylpiperidyl-1)-propyl]-ester und/oder seine pharmakologisch verträglichen Salze, dadurch gekennzeichnet, daß man den (+)-1,4-Dihydro-2,6-dimethyl-4-(3-nitrophenyl)-pyridin-3,5-dicarbonsäure-3-methyl-5-[3-(4,4-diphenylpiperidyl-1)-propyl]-ester und/oder seine pharmakologisch verträglichen Sazle mit galenischen Hilfsstoffen mischt und in eine für Arzneimittel übliche Formulierung überführt.

Europäisches Patentamt

# EUROPÄISCHER RECHERCHENBERICHT

Nummer der Anmeldung

EP 87104269.3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP - A1 - 0 026 317 (BAYER)<br>* Ansprüche 1,7,8; Zusammenfassung *<br>-- | 1,3,5 | C 07 D 211/90<br>A 61 K 31/44 |
| A | CHEMICAL ABSTRACTS, Band 94, Nr. 5, 2. Februar 1981, Columbus, Ohio USA<br>T.SHIBANUMA et al. "Synthesis of optically active 2-(N-benzyl-N--methylamino)ethyl methyl 2,6--dimethyl-4-(m-nitrophenyl)-1,4--dihydro-pyridine-3,5-dicarboxylate"<br>Seite 549, Spalte 2, Zusammenfassung Nr. 30 523f<br>& Chem. Pharm. Bull., 28(9), 2809-12 (1980)<br>-- | 1,3,5, 6 | |
| A | EP - A2/A3 - 0 138 505 (TAKEDA)<br>* Ansprüche 1,12; Zusammenfassung *<br>---- | 1,5 | RECHERCHIERTE SACHGEBIETE (Int. Cl.4)<br>C 07 D 211/00 |

Der vorliegende Recherchenbericht wurde für alle Patentansprüche erstellt.

| Recherchenort | Abschlußdatum der Recherche | Prüfer |
|---|---|---|
| WIEN | 23-06-1987 | HOCHHAUSER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument